# EUROPEAN PATENT APPLICATION

(11) **EP 1 671 599 A1**
(43) Date of publication of application: **21.06.2006**
(21) Application number: 04405771.9
(22) Date of filing: 14.12.2004
(51) Int. Cl.: A61B 17/44

(54) **Obstetric delivery device**

(71) Applicant: Medela Holding AG, 6340 Baar (CH)
(72) Inventor: Uddenberg, Erik, 411 31 Gothenburg (SE)
(74) Representative: Clerc, Natalia

(57) **Abstract**

An obstetric delivery device for Caesarian section is a suction cup for a vacuum extractor and comprises a cup member (1) and a neck portion (2), wherein the suction member has a suction opening (10) intended to be applied to a baby's head prior to delivery and an evacuation opening (11) being connected with an evacuation channel (20) extending through the neck portion (2), wherein the neck portion (2) comprises a handle (3) in the shape of a circumferential flange. This device does not affect the size or position of the skin or uterine incisions. It allows a gentle delivery of the fetal head withoud the need of forceps. It is flexible and atraumatic, with no damage to the mother or the baby. Furthermore it is small and light and easier than forceps to maneuver into position at Caesarion sections. The technique can be easily mastered and is intuitive. Last but not least it allows a fetal head delivery without delay.

## Description

### Background of the invention

WO 89/06112 discloses an obstetric suction device for a vaginal vacuum extractor which is used to assist women during parturition. The device consists of a cup member intended to be applied to the baby's head prior to delivery and a neck portion. The cup member has an opening which communicates with a suction channel extending through the neck portion. This communication channel is connected to a suction pump of the vacuum extractor.

The neck portion has a circumferential flange serving as an attachment for a string. This string is laid around the neck portion behind the flange and is held in grooves therein. The string is at its opposite end attached to a handle. This handle is used as traction device to assist the mother during the vaginal delivery.

Another way of delivering a baby is the well-known Caesarian section. In the Caesarian section the baby is usually moved by hand and no additional tools are used to pull out the baby. The physician must act very quickly so as not to harm the mother or the baby. If elective sections are done the head of the baby may not be engaged. In this case usually Wringley's forceps are used to deliver the fetal head. However, it is often difficult to properly ascertain the precise head position which leads to traction and to a suboptimal appliance of the forceps.

### Summary of the invention

It is therefore an object of the invention to provide a delivery device for Caesarian section which helps the physician to deliver the baby in an optimal manner.

This object is achieved by an obstetric suction device for Caesarian section wherein the device is a suction cup for a vacuum extractor and wherein the device comprises a cup member and a neck portion, wherein the suction member has a suction opening intended to be applied to a baby's head prior to delivery and an evacuation opening being connected with an evacuation channel extending through the neck portion, wherein the neck portion comprises a handle in the shape of a circumferential flange.

The invention is based on the recognition that the obstetric suction device used in vaginal delivery can also be used in the Caesarian section if means are provided for the physician to use the device directly by hand. This means according to the invention is a handle at the neck portion of the cup. By arranging a handle at the neck portion, which is preferably quite short, the device is stiff enough to enable a secure handling and the physician's hand is still near the baby's head.

The inventive device enables a delivery with Caesarian section in a minimum of time. It is generally not difficult to identify the anterior and posterior fontanelle which are readily apparent when the uterine incision is made. Applying the inventive device obviates therefore the need for applying a forceps to deliver the fetal head at Caesarian section and it allows gentle and controlled delivery of the baby. An additional advantage is that no variation of the conventional Caesarian operative technique is required when using the inventive device.

Additional preferred embodiments are described in the dependent claims.

### Brief description of the drawings

The invention will below be described in more detail with reference to a preferred embodiment disclosed in the accompanying drawings.
- Figure 1: shows a side view of a suction device according to the invention and
- Figure 2: shows a vertical section through the device according to figure 1.

### Description of a preferred embodiment

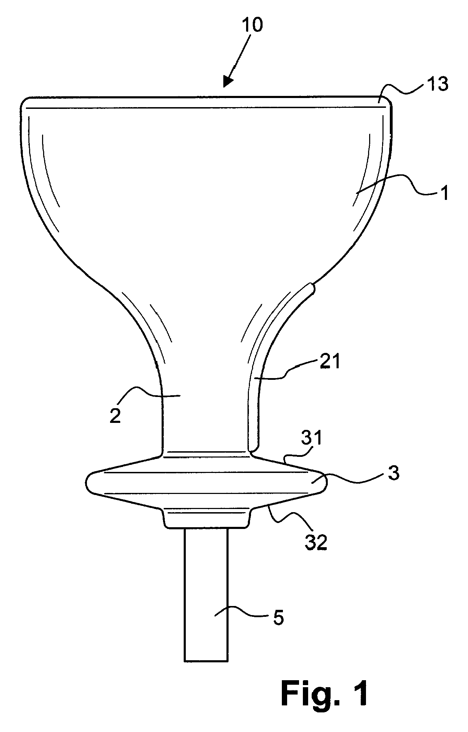

Figure 1 shows an obstetric delivery device according to the invention. It is a suction cup for a vacuum extractor. The vacuum extractor is the same as used with suction cups for vaginal vacuum delivery and since it is well known in the art it is not described here in detail.

The inventive suction cup comprises a cup member 1, a neck portion 2 being connected with the cup member 1 and a handle 3 in the shape of a circumferential flange which is arranged at the neck portion 2 and which surrounds it. The handle 3 is preferably arranged at the end of the neck portion 2 being distant from the cup member 1.

The suction cup, or at least the cup member 1, is preferably made of soft, flexible material, such as silicone rubber. The suction cup is flexible and can be compressed for insertion. Preferably, the cup member 1, the neck portion 2 and the handle 3 are forming a one-piece part, wherein the neck portion 2 and the handle 3 can be stiffer than the cup member 1.

The cup member 1 has a suction opening 10 surrounded by a circumferential thick lip 13. This suction opening 10 is applied to the baby's head. On the opposite end of the cup member 1 an evacuation opening 11 is provided which opens the cup member 1 to an evacuation channel 20. This evacuation channel 20 extends through the neck portion 2 and leads to a connecting pipe 5. This connecting pipe 5 is used to connect the suction cup to a suction line connected with the vacuum extractor.

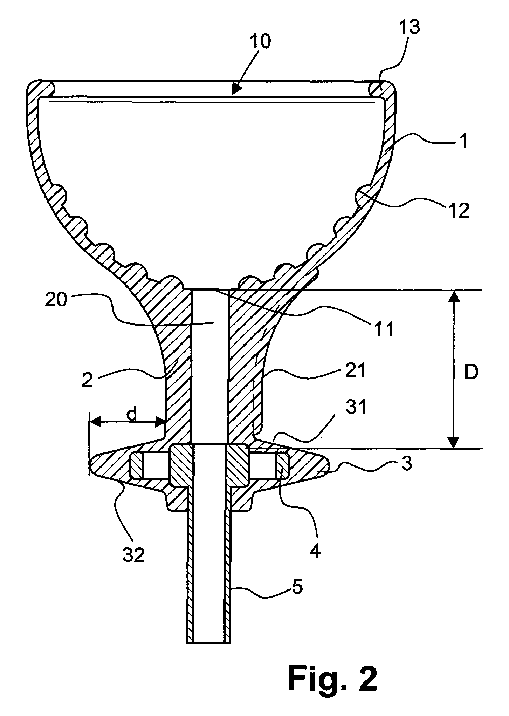

The connecting pipe 5 is preferably made of metal but it is moulded into the handle 3. Furthermore, a reinforcement device, here a metallic ring 4 is moulded into the handle 3 and connected with the connecting pipe 5. This is shown in figure 2. Preferably, the connecting pipe 5 and the metallic ring 4 are a one piece part. The metallic ring can have holes. This stabilizes the handle 3 without making the entire device too heavy.

The cup member 1 preferably comprises on its inner surface a plurality of beads or knobs 12 which are distributed over the inner surface of the suction cup but are distributed at least around the evacuation opening 11.

The neck portion 2 preferably comprises a rib 21 which extends along a peripheral length of the neck portion 2 and which can be used as a position indicator.

The inventive handle 3 comprises an outer rim. This outer rim has preferably a distance d from the neck portion 2 of approximately 16 mm. The distance D between the outer surface 31 of the handle 3 which is directed to the cup member 1 and the evacuation opening 11 of this cup member 1 is preferably 20 to 40 mm, preferably approximately 30 mm. At least the outer surface 31 which is directed to the cup member 1 is approximately flat, wherein the surface can be arranged at an angle to the longitudinal direction of the evacuation channel 20. The opposite outer surface 32 is preferably also flat but runs in an angle to the first surface 31. The distance between the two surfaces 31, 32 is getting smaller in direction to the circumferential rim of the flange 3. This shape helps to optimise the grip of the hand holding the inventive device.

This device is intended to be used in Caesarian sections. The Caesarian section is proceeded in routine fashion until the lower uterine segment is opened. Then the fetal head is elevated cephalad to allow space from the vacuum cup application. The vacuum cup is compressed between the thumb and forefinger and is introduced into the lower segment. The cup is snugly positioned over the fetal occiput preferably in the region of the posterior fontanelle. The electric pump of the vacuum extractor is switched on and a vacuum of about 0.8 kg/cm² is selected. It is not necessary to stepwise increase the vacuum since the modus operandi of this device does not rely on the formation of a chignon. The neck portion 2 is hold between the thumb and the forefinger wherein the handle 3 acts as a handle and a gentle but firm traction is manually applied to maneuver the fetal head outwards until it is delivered. The pump is switched of, the vacuum released and the cup is removed.

This device does not affect the size or position of the skin or uterine incisions. It allows a gentle delivery of the fetal head withoud the need of forceps. It is flexible and atraumatic, with no damage to the mother or the baby. Furthermore it is small and light and easier than forceps to maneuver into position at Caesarion sections. The technique can be easily mastered and is intuitive. Last but not least it allows a fetal head delivery without delay.

### List of reference numbers

- 1: cup member
- 10: suction opening
- 11: evacuation opening
- 12: knobs
- 13: lip
- 2: neck portion
- 20: evacuation channel
- 21: rib
- 3: handle
- 31: first surface
- 32: second surface
- 4: metallic ring
- 5: connecting pipe
- d: distance between outer rim and neck portion
- D: distance between outer surface of handle and evacuation opening

## Claims

1. Obstetric delivery device for Caesarian section wherein the device is a suction cup for a vacuum extractor and wherein the device comprises a cup member (1) and a neck portion (2), wherein the suction member has a suction opening (10) intended to be applied to a baby's head prior to delivery and an evacuation opening (11) being connected with an evacuation channel (20) extending through the neck portion (2), wherein the neck portion (2) comprises a handle (3) in the shape of a circumferential flange.

2. Device according to claim 1 wherein the handle (3) has an outer rim which has a distance from the neck portion of approximately 16 mm.

3. Device according to one of claims 1 or 2 wherein the distance between an outer surface (31) of the handle (3) which is directed to the cup member (1) and the evacuation opening (11) of this cup member (1) is 20 to 40 mm, preferably approximately 30 mm.

4. Device according to one of claims 1 to 3 wherein the handle (3) is reinforced by a metallic ring (4).

5. Device according to one of claims 1 to 4 wherein at least the cup member (1) is made of a flexible material.

6. Device according to one of claims 1 to 5 wherein the cup member (1) comprises an interior side provided with a plurality of beads (12) arranged at a distance from each other and distributed over the inner surface of the cup member (1).

7. Device according to one of claims 1 to 6 wherein the cup member (1), the neck portion (2) and the handle (3) are made as an one-piece part.

8. Device according to one of claims 1 to 7 wherein the neck portion (2) is stiffer than the cup member (1).

9. Device according to one of claims 1 to 8 wherein the handle (3) has at least one flat surface (31), this surface (31) being directed to the cup member (1).

10. Device according to claim 9 wherein the handle (3) has two opposite flat surfaces (31, 32), wherein a first (31) of these two surfaces is directed to the cup member and a second (32) of these two surfaces runs at an angle to the first surface (31).
